# EUROPÄISCHE PATENTSCHRIFT

(11) **EP 0 735 982 B1**
(45) Veröffentlichungstag und Bekanntmachung des Hinweises auf die Patenterteilung: **21.04.1999**
(21) Anmeldenummer: 95904453.8
(22) Anmeldetag: 10.12.1994
(51) Int. Cl.: C01B 15/037, C08K 5/14, C08K 3/20, C07C 407/00, C07C 409/24, C07C 409/26

(54) **VERFAHREN ZUR HERSTELLUNG VON C 1- BIS C 4-MONOPERCARBONSÄURE- UND C 4- BIS C 18-DIPERCARBONSÄURE-KOMPLEXEN IM WIRBELSCHICHTVERFAHREN**
PROCESS FOR PRODUCING C 1 TO C 4 MONOPERCARBOXYLIC ACID AND C 4 TO C 18 DIPERCARBOXYLIC ACID COMPLEXES BY THE FLUIDISED BED PROCESS
PROCEDE DE FABRICATION DE COMPLEXES D'ACIDES MONOPERCARBOXYLIQUES C 1 A C 4 ET D'ACIDES DIPERCARBOXYLIQUES C 4 A C 18 SELON LA TECHNIQUE EN LIT FLUIDISE

(30) Priorität: 23.12.1993 DE 4344131
(43) Veröffentlichungstag der Anmeldung: 09.10.1996
(73) Patentinhaber: BASF AKTIENGESELLSCHAFT, 67056 Ludwigshafen (DE)
(72) Erfinder: BREITENBACH, Jörg, D-53545 Linz (DE); GRABOWSKI, Sven, D-67061 Ludwigshafen (DE); SANNER, Axel, D-67227 Frankenthal (DE)
(86) Internationale Anmeldenummer: EP9404115
(87) Internationale Veröffentlichungsnummer: WO9517345

(56) Entgegenhaltungen:
- WO-A-92/17158
- US-A- 3 480 557
- US-A- 5 190 749

## Beschreibung

Die vorliegende Erfindung betrifft ein verbessertes Verfahren zur Herstellung von C₁- bis C₄-Monopercarbonsäure- und C₄- bis C₁₈-Dipercarbonsaure-Komplexen im Wirbelschichtverfahren durch Aufbringen einer Lösung von C₁- bis C₄-Monopercarbonsäuren, C₄- bis C₁₈-Dipercarbonsäuren oder eine Mischung hieraus in Wasser oder Carbonsäuren auf eine pulverförmige oder vorgranulierte Matrix in oder außerhalb der Wirbelschicht und gleichzeitige oder anschließende Wirbelschichttrocknung.

Da ein Teil der nach dem erfindungsgemäßen Verfahren hergestellten Komplexe neue Stoffe darstellen, betrifft die Erfindung weiterhin diese neuen Stoffe.

Aus der US-A 3 376 110 (1) und der US-A 3 480 557 (2) sind wasserunlösliche (vernetzte) bzw. wasserlösliche (unvernetzte) Wasserstoffperoxid-Komplexe von polymeren N-Vinylheterocyclen wie Poly-N-vinylpyrrolidon und auch Poly-N-vinyl-2-caprolactam bekannt. Diese Komplexe können durch Eindampfen einer waßrigen Suspension aus Polymerisat und Wasserstoffperoxid hergestellt werden.

In der US-A 5 077 047 (3) wird ein Verfahren zur Herstellung von frei fließenden pulverförmigen Wasserstoffperoxid-Poly-N-vinylpyrrolidon-Komplexen im Wirbelschichtverfahren beschrieben. Die Trocknung des gebildeten Produktes kann in der Wirbelschicht gleichzeitig oder in einer nachgeschalteten Stufe erfolgen.

Aus der US-A 5 190 749 (4) ist bekannt, daß man das Wirbelschichtverfahren aus (3) auch für die Herstellung von Wasserstoffperoxid-Komplexen aus Copolymerisaten von N-Vinylpyrrolidon mit quaternisierten Ammonium-Monomeren einsetzen kann.

Auch Wasserstoffperoxid-Harnstoff-Komplexe lassen sich gemäß der DE-A 34 44 552 (5) in einem Wirbelschichtverfahren herstellen, wobei die Trocknung des Reaktionsproduktes dabei in einem Arbeitsgang in der gleichen Apparatur oder in einer nachgeschalteten Trocknungsstufe erfolgen kann.

Aus der WO 92/17158 (7) ist bekannt, daß man als Schaumporenvergrößerungsmittel eine Hydroxyalkylcellulose wie Hydroxyethylcellulose den Wasserstoffperoxid-Poly-N-vinylpyrrolidon-Komplexen bei der Herstellung im Wirbelbett zusetzen kann.

Da die im Stand der Technik beschriebenen Herstellungsverfahren für derartige Wasserstoffperoxid-Komplexe noch nicht ausreichend effizient und wirtschaftlich arbeiten, lag der vorliegenden Erfindung die Aufgabe zugrunde, ein verbessertes Herstellungsverfahren bereitzustellen. Außerdem weisen die bekannten Wasserstoffperoxid-Polymerisat-Komplexe Mängel hinsichtlich ihrer Eigenschaften bei der Herstellung und bei der Anwendung auf, so daß auch ein Bedürfnis nach neuen verbesserten Komplexen von Perverbindungen besteht.

Demgemäß wurde das eingangs definierte Verfahren zur Herstellung von C₁- bis C₄-Monopercarbonsäure- und C₄- bis C₁₈-Dipercarbonsäure-Komplexen im Wirbelschichtverfahren gefunden, welches dadurch gekennzeichnet ist, daß man als Matrix
A) N-Vinylcaprolactam-Homopolymerisate,
B) N-Vinylcaprolactam-Copolymerisate mit N-Vinylpyrrolidon, N-Vinylimidazol, Acryl- oder Methacrylamidopropyl-3-sulfonsäure, Acryl- oder Methacrylsaure oder Gemischen hieraus als Comonomeren im Gewichtsverhältnis N-Vinylcaprolactam zu Comonomere von 20:1 bis 1:20,
C) N-Vinylpyrrolidon-Copolymerisate mit N-Vinylimidazol, Acryl- oder Methacrylsäure oder Gemischen hieraus als Comonomeren im Gewichtsverhältnis N-Vinylpyrrolidon zu Comonomere von 20:1 bis 1:20,
D) N-Vinylimidazol-Homopolymerisate, welche am heterocyclischen 5 Ring durch bis zu drei C₁- bis C₄-Alkylreste substituiert sein und in durch C₁- bis C₄-Alkyl N-quaternisierter Form vorliegen können,
E) Mischungen aus Monosacchariden, Oligosacchariden, Polysacchariden oder Derivaten hiervon mit den Polymerisaten A bis D im Gewichtsverhältnis von 1:99 bis 90:10,
F) Mischungen aus Monosacchariden, Oligosacchariden, Starken, Starkeabbauprodukten oder Derivaten hiervon mit N-Vinylpyrrolidon-Homopolymerisaten im Gewichtsverhältnis 1 : 99 bis 90 : 10,
G) Mischungen aus Trehalose, Saccharose, Glucose, α-, β- oder γ-Cyclodextrinen oder Derivaten dieser Cyclodextrine oder Gemischen hieraus mit den Polymerisaten A bis D oder mit N-Vinylpyrrolidon-Homopolymerisaten im Gewichtsverhältnis von 1:99 bis 100:0 oder
H) für den Fall von C₁- bis C₄-Monopercarbonsäure- und C₄- bis C₁₈-Dipercarbonsäure-Komplexen N-Vinylpyrrolidon-Homopolymerisate

### einsetzt.

Unter N-Vinylcaprolactam ist N-Vinyl-ε-caprolactam (N-Vinyl-2-caprolactam) zu verstehen. Unter N-Vinylpyrrolidon ist N-Vinyl-2-pyrrolidon zu verstehen.

Als Copolymerisate B auf Basis von N-Vinylcaprolactam kommen vor allem N-Vinylcaprolactam-N-Vinylpyrrolidon-Bispolymerisate, N-Vinylcaprolactam-N-Vinylimidazol-Bispolymerisate, N-Vinylcaprolactam-N-Vinylpyrrolidon-N-Vinylimidazol-Terpolymerisate, N-Vinylcaprolactam-Acryl- oder Methacrylsäure-Bispolymerisate und N-Vinylcaprolactam-Acryl- oder Methacrylamidopropyl-3-sulfonsäure-Bispolymerisate in Betracht. Die gegebenenfalls vorliegenden Carbonsaure- oder Sulfonsaure-Gruppen können dabei teilweise oder vollständig als Salze vorliegen, z.B. als Natrium-, Kalium-, Ammonium- oder substituierte Ammoniumsalze.

Die Polymerisate B und auch die Hompolymerisate A können weiterhin unvernetzt oder vernetzt sein. Durch Vernetzung kann der Grad der Wasserlöslichkeit eingestellt werden. Das erfindungsgemaße Verfahren läßt sich besonders gut mit vernetzten N-Vinylcaprolactam-N-Vinylpyrrolidon-Copolymerisaten ("Popcorn"-Polymerisaten) gemäß DE-C 24 37 629 (6) durchführen. Zur Vernetzung setzt man bei der Polymerisation am besten Divinylethylenharnstoff als vernetzendes Comonomeres in Mengen von 0,5 bis 10 Gew.-%, vorzugsweise 2 bis 8 Gew.-%, bezogen auf das Gewicht der unter B genannten Monomeren, zu. Man kann aber auch die Vernetzung durch Zugabe von Katalysatoren wie Alkalimetallhydriden oder -borhydriden bewirken.

Bei den Copolymerisaten B beträgt das Gewichtsverhältnis von N-Vinylcaprolactam zu den Comonomeren N-Vinylpyrrolidon, N-Vinylimidazol, Acryl- oder Methacrylamidopropyl-3-sulfonsaure und/oder Acryl- oder Methacrylsaure 20:1 bis 1:20, vorzugsweise 15:1 bis 1:4, insbesondere 10:1 bis 1:1.

Als Copolymerisate C auf Basis von N-Vinylpyrrolidon kommen vor allem N-Vinylpyrrolidon-N-Vinylimidazol-Bispolymerisate und N-Vinylpyrrolidon-Acrylsäure- oder Methacrylsäure-Bispolymerisate in Betracht. Die Copolymerisate C können in Analogie zu den Polymerisaten A und B unvernetzt oder vernetzt sein.

Bei den Copolymerisaten C beträgt das Gewichtsverhältnis von N-Vinylpyrrolidon zu den Comonomeren N-Vinylimidazol und/oder Acryl- oder Methacrylsäure 20:1 bis 1:20, vorzugsweise 4:1 bis 1:10, insbesondere 1:1 bis 1:8.

Als durch bis zu drei, insbesondere durch bis zu zwei, vor allem durch einen C₁- bis C₄-Alkylrest substituierte N-Vinylimidazol-Homopolymerisate D kommen beispielsweise solche des 2-Methyl-, 4-Methyl- oder 5-Methylimidazols in Betracht.

Als N-Vinyl-N'-C₁- bis C₄-alkyl-imidazolium-Homopolymerisate D können beispielsweise N-Vinyl-N'-methyl-, N-Vinyl-N'-ethyl- oder N-Vinyl-N'-butyl-imidazolium-Homopolymerisate eingesetzt werden. Als Gegenionen zu den kationischen quaternären Stickstoffatomen können z.B. Chlorid, Bromid oder Methosulfat auftreten. Die Quaternierung der Stickstoffatome kann vor oder nach der Polymerisation erfolgt sein. Die Homopolymerisate D können in Analogie zu den Polymerisaten A bis C unvernetzt oder vernetzt sein.

Mischungen aus den Polymerisaten A bis D oder N-Vinylpyrrolidon-Homopolymerisaten mit Kohlenhydraten oder Kohlenhydrat-Derivaten gemäß E und F zeigen besonders gute Anwendungseigenschaften, insbesondere ist das Auflösevermögen der fertigen Wasserstoffperoxid- bzw. Percarbonsäure-Komplexe optimal. Das Gewichtsverhältnis der Kohlenhydrate bzw. Kohlenhydrat-Derivate zu den genannten Polymerisaten beträgt hierbei 1:99 bis 90:10, vorzugsweise 10:90 bis 70:30, insbesondere 20:80 bis 50:50.

Bei der Kohlenhydrat-Komponente handelt es sich um Naturstoffe auf der Basis von Monosacchariden, Oligosacchariden, Polysacchariden oder deren Derivaten. Vom wirtschaftlichen Standpunkt aus gesehen verwendet man bei der Herstellung vorzugsweise Stärken, thermisch und/oder mechanisch behandelte Starken, oxidativ, hydrolytisch oder enzymatisch abgebaute Stärken, oxidierte hydrolytisch oder oxidierte enzymatisch abgebaute Stärken sowie chemisch modifizierte Stärken und chemisch modifizierte Monosaccharide und Oligosaccharide. Prinzipiell sind alle Stärken geeignet. Die Stärken sind praktisch nicht in Wasser löslich und können in bekannter Weise durch thermische und/oder mechanische Behandlung oder durch einen enzymatischen oder einen säurekatalysierten Abbau in eine wasserlösliche Form übergeführt werden.

Als Starkeabbauprodukte, die entweder durch oxidativen, hydrolytischen oder enzymatischen Abbau von Starke erhältlich sind, seien beispielsweise folgende Verbindungen genannt: Dextrine wie Weiß- und Gelbdextrine, Maltodextrine, Glucosesirup, Maltosesirup, Hydrolysate mit hohem Gehalt an D-Glucose sowie Maltose und D-Glucose und deren Isomerisierungsprodukt Fructose. Als Komponente B eignen sich auch Mono- und Oligosaccharide wie Galaktose, Mannose, Ribose, Saccharose, Raffinose, Laktose, Trehalose sowie Abbauprodukte der Cellulose, beispielsweise Cellubiose und ihre Oligomeren. Weiterhin eignen sich die Cyclodextrine verschiedener Ringgröße und deren Derivate.

### Bevorzugt werden nicht reduzierende Saccharide.

Setzt man gemäß G als Kohlenhydrat-Komponente Trehalose, Saccharose, Glucose, α-, β- oder γ-Cyclodextrine oder Derivate dieser Cyclodextrine oder Gemische hieraus ein, kann deren Anteil im Matrix-Material bis zu 100 Gew.-% erhöht werden, d.h. man kann beim erfindungsgemäßen Verfahren die Perverbindung sogar auf die reinen Kohlenhydrate aufbringen. Das Gewichtsverhältnis dieser Kohlenhydrat-Komponenten zu den Polymerisaten A bis D bzw. N-Vinylpyrrolidon-Homopolymerisaten kann also über E bzw. F hinausgehend 1:99 bis 100:0, vorzugsweise 10:90 bis 100:0, insbesondere 20:80 bis 100:0 betragen.

Die K-Werte (nach Fikentscher) als Maß für das Molekulargewicht der eingesetzten Polymerisate A bis D und H liegen meist bei 10 bis 90, insbesondere 17 bis 60, gemessen unter den hierfür üblichen Bedingungen, z.B. in H₂O bei Raumtemperatur.

Man kann bei der Herstellung der Polymerisate A bis D und H bzw. der den Mischungen E bis G zugrundeliegenden Polymerisate in geringen Mengen weitere übliche copolymerisierbare Monomere hinzufügen, um die Eigenschaften dieser Polymerisate im Rahmen der vorliegenden Erfindung geringfügig zu modifizieren.

Als C₁- bis C₄-Monopercarbonsäuren (Monoperoxycarbonsäuren) eignen sich vor allem C₂- bis C₄-Monopercarbonsäuren, insbesondere Peressigsäure, Perpropionsäure, Perbuttersaure und Isoperbuttersäure. Auch Perameisensäure kann verwendet werden, wenn die notwendigen Sicherheitsvorkehrungen im Hinblick auf die Instabilität dieser Verbindung getroffen werden.

Als C₄- bis C₁₈-Dipercarbonsäuren (Diperoxycarbonsäuren) eignen sich vor allem C₈- bis C₁₄-Dipercarbonsäuren, insbesondere 1,12-Diperoxydodecansäure und 1,14-Diperoxytetradecansäure.

Man setzt Lösungen der Perverbindungen mit einem Gehalt von normalerweise 0,5 bis 30 Gew.-%, insbesondere 1 bis 15 Gew.-% in Wasser, in Carbonsäuren, meist den zugrundeliegenden Carbonsäuren, oder Mischungen aus Wasser und Carbonsäuren ein. Bei Verwendung von Percarbonsauren enthalten waßrige Lösungen meist noch zusätzlich Wasserstoffperoxid.

Das Aufbringen der waßrigen Lösungen der Perverbindungen auf die pulverförmige oder vorgranulierte Matrix A bis H erfolgt vorzugsweise in der Wirbelschicht, kann aber auch in einem vorgeschalteten Schritt vorgenommen werden. Das Aufbringen erfolgt zweckmäßigerweise durch Versprühen der Lösungen mittels Düsen.

Die Wirbelschichttrocknung zur Entfernung des hauptsächlich durch die wäßrigen Lösungen der Perverbindungen eingebrachten Wassers wird vorzugsweise gleichzeitig mit dem Aufbringen der Perverbindungen auf die Matrix vorgenommen, kann aber auch in einem separaten Schritt in derselben oder in einer nachgeschalteten Apparatur durchgeführt werden. Um ein freifließendes, d.h. rieselfähiges Pulver der Wasserstoffperoxid- bzw. Percarbonsäure-Komplexe zu erhalten, sollte das Wassergehalt kleiner als 5 Gew.-% nach der Trocknung betragen; ein Verblocken wird so vermieden.

Die Temperatur beim Aufbringen der wäßrigen Lösungen der Perverbindungen liegt üblicherweise bei der Zuluft bei 25 bis 80°C und bei der Abluft bei 25 bis 70°C, bevorzugt wird für Zu- und Abluft ein Temperaturbereich von jeweils 30 bis 60°C, insbesondere 35 bis 55°C. Weitere Einzelheiten zur Durchführung eines derartigen Wirbelschicht- (Wirbelbett) -Verfahrens bezüglich theoretischer Grundlagen, Reaktortypen und technischer Ausführungsformen können Ullmanns Encyklopädie der technischen Chemie, 4. Auflage, Band 3 (1973), S. 433-460 und S. 480-493 entnommen werden.

Die nach den erfindungsgmäßen Verfahren hergestellten Percarbonsäure-Komplexe weisen Gehalte an Perverbindungen von 1 bis 35 Gew.-%, vorzugsweise 3 bis 30 Gew.-%, insbesondere 5 bis 25 Gew.-%, bezogen auf den Feststoffanteil im 5 erhaltenen Produkt, auf.

Die beschriebenen Percarbonsäure-Komplexe lassen sich prinzipiell auch problemlos und mit denselben Ausbeuten, Reinheiten und Gehalten an Perverbindungen durch Ausfällen aus wäßrigen Lösungen der Polymerisate A bis D und H durch Zugabe wäßriger Lösungen der Perverbindungen, Abfiltrieren oder Abdekantieren und Trocknen erhalten, nur ist diese Technik nicht mit befriedigenden Ergebnissen auf den großtechnischen Maßstab übertragbar.

Gegenstand der vorliegenden Erfindung sind auch C₁- bis C₄-Monopercarbonsäure- und C₄- bis C₁₈-Dipercarbonsäure-Komplexe von N-Vinylcaprolactam-Copolymerisaten mit N-Vinylpyrrolidon, N-Vinylimidazol, Acryl- oder Methacrylamidopropyl-3-sulfonsäure, Acryl- oder Methacrylsaure oder Gemischen hieraus als Comonomeren im Gewichtsverhältnis N-Vinylcaprolactam zu Comonomere von 20:1 bis 1:20 mit einem Gehalt von 1 bis 35 Gew.-% an Perverbindungen.

Gegenstand der vorliegenden Erfindung sind weiterhin C₁- bis C₄-Monopercarbonsäure- und C₄- bis C₁₈-Dipercarbonsäure-Komplexe von N-Vinylpyrrolidon-Copolymerisaten mit N-Vinylimidazol, Acryl- oder Methacrylsäure oder Gemischen hieraus als Comonomeren im Gewichtsverhältnis N-Vinylpyrrolidon zu Comonomeren von 20:1 bis 1:20 mit einem Gehalt von 1 bis 35 Gew.-% an Perverbindungen.

Gegenstand der vorliegenden Erfindung sind weiterhin C₁- bis C₄-Monopercarbonsäure- und C₄- bis C₁₈-Dipercarbonsäure-Komplexe von N-Vinylimidazol-Homopolymerisaten, welche am heterocyclischen Ring durch bis zu drei C₁- bis C₄-Alkylreste substituiert sein und in durch C₁- bis C₄-Alkyl N-quaternisierter Form vorliegen können, mit einem Gehalt von 1 bis 35 Gew.-% an Perverbindungen.

Gegenstand der vorliegenden Erfindung sind weiterhin C₁- bis C₄-Monopercarbonsäure- und C₄- bis C₁₈-Dipercarbonsäure-Komplexe von Mischungen aus Monosacchariden, Oligosacchariden, Polysacchariden oder Derivaten hiervon mit
(a) N-Vinylcaprolactam-Homopolymerisaten,
(b) N-Vinylcaprolactam-Copolymerisaten mit N-Vinylpyrrolidon, N-Vinylimidazol, Acryl- oder Methacrylamidopropyl-3-sulfonsäure, Acryl- oder Methacrylsäure oder Gemischen hieraus als Comonomeren im Gewichtsverhältnis N-Vinylcaprolactam zu Comonomere von 20:1 bis 1:20,
(c) N-Vinylpyrrolidon-Copolymerisaten mit N-Vinylimidazol, Acryl- oder Methacrylsäure oder Gemischen hieraus als Comonomeren im Gewichtsverhältnis N-Vinylpyrrolidon zu Comonomere von 20:1 bis 1:20 oder
(d) N-Vinylimidazol-Homopolymerisaten, welche am heterocyclischen Ring durch bis zu drei C₁- bis C₄-Alkylreste substituiert sein und in durch C₁- bis C₄-Alkyl N-quaternisierter Form vorliegen können,
im Gewichtsverhältnis von 1:99 bis 90:10 mit einem Gehalt von 1 bis 35 Gew.-% an Perverbindungen.

Gegenstand der vorliegenden Erfindung sind weiterhin C₁- bis C₄-Monopercarbonsäure- und C₄- bis C₁₈-Dipercarbonsäure-Komplexe von Mischungen aus Monosacchariden, Oligosaccharide, Stärken, Starkeabbauprodukten oder Derivaten hiervon mit N-Vinylpyrrolidon-Homopolymerisaten im Gewichtsverhältnis von 1 : 99 bis 90 : 10 mit einem Gehalt von 1 bis 35 Gew.-% an Perverbindungen.

Gegenstand der vorliegenden Erfindung sind weiterhin C₁- bis C₄-Monopercarbonsäure- und C₄- bis C₁₈-Dipercarbonsäure-Komplexe von Mischungen aus Trehalose, Saccharose, Glucose, α-, β- oder γ-Cyclodextrine oder Mischungen dieser Cyclodextrine oder Gemischen hieraus mit
(a) N-Vinylcaprolactam-Homopolymerisaten,
(b) N-Vinylcaprolactam-Copolymerisaten mit N-Vinylpyrrolidon, N-Vinylimidazol, Acryl- oder Methacrylamidopropyl-3-sulfonsäure, Acryl- oder Methacrylsäure oder Gemischen hieraus als Comonomeren im Gewichtsverhältnis N-Vinylcaprolactam zu Comonomere von 20:1 bis 1:20,
(c) N-Vinylpyrrolidon-Copolymerisaten mit N-Vinylimidazol, Acryl- oder Methacrylsäure oder Gemischen hieraus Comonomeren im Gewichtsverhältnis N-Vinylpyrrolidon zu Comonomere von 20:1 bis 1:20,
(d) N-Vinylimidazol-Homopolymerisaten, welche am heterocyclischen Ring durch bis zu drei C₁- bis C₄-Alkylreste substituiert sein und in durch C₁- bis C₄-Alkyl N-quaternisierter Form vorliegen können, oder
(e) N-Vinylpyrrolidon-Homopolymerisaten im Gewichtsverhaltnis von 1:99 bis 100:0 mit einem Gehalt von 1 bis 35 Gew.-% an Perverbindungen.

Gegenstand der vorliegenden Erfindung sind weiterhin C₁- bis C₄-Monopercarbonsäure- und C₄- bis C₁₈-Dipercarbonsäure-Komplexe von N-Vinylpyrrolidon-Homopolymerisaten mit einem Gehalt von 1 bis 35 Gew.-% an Perverbindungen.

Mit dem erfindungsgemäßen Verfahren ist es möglich, Percarbonsäure-Komplexe verschiedener Hydrophilie in Abhängigkeit vom Anteil an hydrophobem Comonomer wie N-Vinylcaprolactam in effizienter und wirtschaftlicher Weise zu erhalten. Insbesondere bei Matrixmaterial auf der Basis von N-Vinylcaprolactam verläuft die Wirbelschichttrocknung schneller als bei N-Vinylpyrrolidon-Homopolymerisaten, da die Hygroskopizität geringer ist; solche Pulver sind frei fließend. Bei der Wärmelagerung der mittels der Matrixmaterialen A bis H hergestellten Komplexe der Perverbindungen erzielen die erfindungsgemäß hergestellten Produkte in der Regel bessere Ergebnisse bei der Stabilitätsuntersuchung (beispielsweise mittels Differentialthermogravimetrie) als die Produkte des Standes der Technik.

Aus der Differentialthermogravimetrie (Heizrate von 2°K/min, 30°-400°C) ergibt sich für einen nicht vernetzten Polyvinylcaprolactam-H₂O₂-Komplex mit 4,8 Gew.-% H₂O₂ eine Onset-Temperatur von 110°C und eine Peakmaximumtemperatur von 165°C. Ein nicht vernetzter handelsüblicher Polyvinylpyrrolidon-H₂O₂-Komplex mit 18 Gew.-% H₂O₂ besitzt dagegen eine Onset-Temperatur von nur 60°C und eine Peakmaximumtemperatur von 155°C.

Nach dem erfindungsgemäßen Verfahren hergestellte Percarbonsäure-Komplexe können beispielsweise Verwendung finden in der Desinfektion oder Konservierung, insbesondere in Zahnpasten, bei der Akne-Therapie, als Wundauflagen, in der Kosmetik, z.B. in der Haarkosmetik (Haarfärbung, Haarbleiche) und in der Depilation, oder als feste Komponente für chemische Reaktionen wie Polymerisationen oder Oxidationen, weiterhin als Waschmittelzusatz oder als Hilfsmittel in der Textil- und Papierbleiche sowie als Bestandteil von Filtersystemen, z.B. für die Wasserbehandlung oder in der Medizin für die Blutbehandlung, in die die beschriebenen Komplexe mit vernetzten Polymeren eingearbeitet oder aufgebracht werden können.

### Beispiele

In der Prüfung als Desinfektionsmittel nach den Richtlinien der Deutschen Gesellschaft für Hygiene und Mikrobiologie (DGHM) wurde die minimale Hemmkonzentration bestimmt. Dabei zeigte ein nicht vernetzter handelsüblicher Polyvinylpyrrolidon-H₂O₂-Komplex (20,2 Gew.-% H₂O₂) einen Wert von 5,0 %, während ein nicht vernetzter Polyvinylcaprolactam-H₂O₂-Komplex (10,9 Gew.-% H₂O₂) einen Wert von 5,0 % und ein H₂O₂-Komplex mit einer Mischung aus einem nicht vernetzten Polyvinylpyrrolidon und Maltodextrin (gemäß nachfolgendem Beispiel 4 oder 5) mit einem Gehalt von 10,1 Gew.-% H₂O₂ einen Wert von 2,5 % aufwies.

Geprüft wird hierbei nach den Richtlinien für die Prüfung und Bewertung chemischer Desinfektionsverfahren (Stand: 01.01.1981) der DGHM. Die Auswertung erfolgt nach 72stündiger Bebrütung bei 36°C. Die Verdünnung erfolgt mit Wasser standardisierter Härte ohne weitere Hilfsmittel (Tenside). Die Einstellung des pH-Wertes auf 7,2 wird mit 0,1 mol/l NaOH bzw. mit 0,1 mol/l HCl durchgeführt. Die Abstufung der Prüfkonzentrationen erfolgt gemäß den nachfolgend aufgeführten Verdünnungsschritten (alle Angaben in Vol.-%):
100, 75, 50, 25, 20, 15, 10, 7,5, 5,0, 4,0, 3,0, 2,5, 2,0, 1,5, 1,00, 0,75, 0,50, 0,25, 0,10, 0,05, 0,025, 0,0125, 0,00625 (usw. in geometrischer Reihe, d.h. jeweils um den Faktor 0,5 reduzierte Konzentration).

### Herstellung im Wirbelschichtverfahren

Die Wirbelschichtgranulation wurde in einem Granulierzylinder, der nach unten durch einen Lochboden mit Siebauflage (Maschenweite 10-500 µm) und nach oben durch 4 Filtersäcke, welche alle 15 s durch Druckluft freigeblasen wurden, durchgeführt. Eine Zweistoffdüse mit verlagertem Flüssigkeitseinsatz befand sich 28 cm über dem Siebboden. Die Zudosierung der Wasserstoffperoxid- oder Peressigsäure-Lösung erfolgte über eine Schlauchpumpe. Die Prozeßluft konnte auch durch Stickstoff ersetzt werden. Der Luftdurchsatz betrug ca. 120 m³/h und konnte grob durch eine Abluftklappe reguliert werden. Beim Trocknen wurde der Luftstrom auf 150 m³/h erhöht. Die mögliche Einsatzmenge an festem Polymerpulver für diese Apparatur lag zwischen 100 und 4000 g. Die mittlere Korngröße war durch die beiden Parameter Zugaberate und Luftdruck der Sprühdüse einstellbar.

### Beispiel 1

100 g N-Vinylpyrrolidon-Homopolymerisat (K-Wert: 30, gemessen in H₂O bei 25°C) wurden mit 5 gew.-%iger wäßriger Peressigsäure-Lösung, die 26-27 Gew.-% Wasserstoffperoxid enthielt, bei einer Zulufttemperatur von 40°C und einer Ablufttemperatur von 35°C behandelt und in Analogie zu Beispiel 1 getrocknet.

Tabelle 6 zeigt die Menge an aufgebrachter Persäure-Lösung und die im Festprodukt gefundenen H₂O₂/Persäure-Gehalte:

**Tabelle 6**

| Persäure-Lösung [g] | H₂O₂/Persäure-Gehalt [Gew.-%] |
|---|---|
| 10 | 1,7 / 0,7 |
| 20 | 4,0 / 1,2 |
| 30 | 6,7 / 1,5 |
| 40 | 7,7 / 1,7 |

Der Komplex verlor nach 4 h bei 70°C ca. 10 % des Wasserstoffperoxid-Gehaltes und 12 % des Peressigsauregehaltes.

### Beispiel 2

Ein N-Vinylpyrrolidon-N-Vinylcaprolactam-Copolymerisat im Gewichtsverhältnis von 1:9 (100 g) wurde in Analogie zu Beispiel 9 behandelt.

Tabelle 7 zeigt die Menge an aufgebrachter Persäure-Lösung und die im Festprodukt gefundenen H₂O₂/Persäure-Gehalte:

**Tabelle 7**

| Persäure-Lösung [g] | H₂O₂/Persäure-Gehalt [Gew.-%] |
|---|---|
| 10 | 1,9 / 0,5 |
| 20 | 4,2 / 0,9 |
| 30 | 6,9 / 1,5 |
| 40 | 8,5 / 1,9 |

### Beispiel 3

100 g vernetztes N-Vinylpyrrolidon-Homopolymerisat wurden in Analogie zu Beispiel 9 behandelt.

Tabelle 8 zeigt die Menge an aufgebrachter Persäure-Lösung und die im Festprodukt gefundenen H₂O₂/Persäure-Gehalte:

**Tabelle 8**

| Persäure-Lösung [g] | H₂O₂/Persäure-Gehalt [Gew.-%] |
|---|---|
| 10 | 1,8 / 0,4 |
| 20 | 4,1 / 0,8 |
| 30 | 6,9 / 1,4 |
| 40 | 7,9 / 1,8 |

Der Komplex verlor nach 7 h bei 70°C ca. 11 % des Wasserstoffperoxid-Gehaltes und 10 % des Peressigsauregehaltes.

## Patentansprüche

1. Verfahren zur Herstellung von C₁- bis C₄-Monopercarbonsäure- und C₄- bis C₁₈-Dipercarbonsäure-Komplexen im Wirbelschichtverfahren durch Aufbringen einer Lösung C₁- bis C₄-Monopercarbonsäuren, C₄- bis C₁₈-Dipercarbonsäuren oder einer Mischung hieraus in Wasser oder Carbonsäuren auf eine pulverförmige oder vorgranulierte Matrix in oder außerhalb der Wirbelschicht und gleichzeitige oder anschließende Wirbelschichttrocknung, dadurch gekennzeichnet, daß man als Matrix
A) N-Vinylcaprolactam-Homopolymerisate,
B) N-Vinylcaprolactam-Copolymerisate mit N-Vinylpyrrolidon, N-Vinylimidazol, Acryl- oder Methacrylamidopropyl-3-sulfonsäure, Acryl- oder Methacrylsäure oder Gemischen hieraus als Comonomeren im Gewichtsverhältnis N-Vinylcaprolactam zu Comonomere von 20:1 bis 1:20,
C) N-Vinylpyrrolidon-Copolymerisate mit N-Vinylimidazol, Acryl- oder Methacrylsäure oder Gemischen hieraus als Comonomeren im Gewichtsverhältnis N-Vinylpyrrolidon zu Comonomere von 20:1 bis 1:20,
D) N-Vinylimidazol-Homopolymerisate, welche am heterocyclischen Ring durch bis zu drei C₁- bis C₄-Alkylreste substituiert sein und in durch C₁- bis C₄-Alkyl N-quaternisierter Form vorliegen können,
E) Mischungen aus Monosacchariden, Oligosacchariden, Polysacchariden oder Derivaten hiervon mit den Polymerisaten A bis D im Gewichtsverhältnis von 1:99 bis 90:10,
F) Mischungen aus Monosacchariden, Oligosacchariden, Stärken, Stärkeabbauprodukten oder Derivaten hiervon mit N-Vinylpyrrolidon-Homopolymerisaten im Gewichtsverhältnis 1 : 99 bis 90 : 10,
G) Mischungen aus Trehalose, Saccharose, Glucose, α-, β- oder γ-Cyclodextrinen oder Derivaten dieser Cyclodextrine oder Gemischen hieraus mit den Polymerisaten A bis D oder mit N-Vinylpyrrolidon-Homopolymerisaten im Gewichtsverhältnis von 1:99 bis 100:0 oder
H) für den Fall von C₁- bis C₄-Monopercarbonsäure- und C₄-bis C₁₈-Dipercarbonsäure-Komplexen N-Vinylpyrrolidon-Homopolymerisate
einsetzt.

2. C₁- bis C₄-Monopercarbonsäure- und C₄- bis C₁₈-Dipercarbonsäure-Komplexe von N-Vinylcaprolactam-Copolymerisaten mit N-Vinylpyrrolidon, N-Vinylimidazol, Acryl- oder Methacrylamidopropyl-3-sulfonsäure, Acryl- oder Methacrylsäure oder Gemischen hieraus als Comonomeren im Gewichtsverhältnis N-Vinylcaprolactam zu Comonomere von 20:1 bis 1:20 mit einem Gehalt von 1 bis 35 Gew.-% an Perverbindungen.

3. C₁- bis C₄-Monopercarbonsäure- und C₄- bis C₁₈-Dipercarbonsäure-Komplexe von N-Vinylpyrrolidon-Copolymerisaten mit N-Vinylimidazol, Acryl- oder Methacrylsäure oder Gemischen hieraus als Comonomere im Gewichtsverhältnis N-Vinylpyrrolidon zu Comonomere von 20:1 bis 1:20 mit einem Gehalt von 1 bis 35 Gew.-% an Perverbindungen.

4. C₁- bis C₄-Monopercarbonsäure- und C₄- bis C₁₈-Dipercarbonsäure-Komplexe von N-Vinylimidazol-Homopolymerisaten, welche am heterocyclischen Ring durch bis zu drei C₁- bis 04-Alkylreste substituiert sein und in durch C₁- bis C₄-Alkyl N-quaternisierter Form vorliegen können, mit einem Gehalt von 1 bis 35 Gew.-% an Perverbindungen.

5. C₁- bis C₄-Monopercarbonsäure- und C₄- bis C₁₈-Dipercarbonsäure-Komplexe von Mischungen aus Monosacchariden, Oligosacchariden, Polysacchariden oder Derivaten hiervon mit
(a) N-Vinylcaprolactam-Homopolymerisaten,
(b) N-Vinylcaprolactam-Copolymerisaten mit N-Vinylpyrrolidon, N-Vinylimidazol, Acryl- oder Methacrylamidopropyl-3-sulfonsäure, Acryl- oder Methacrylsäure oder Gemischen hieraus als Comonomeren im Gewichtsverhältnis N-Vinylcaprolactam zu Comonomere von 20:1 bis 1:20,
(c) N-Vinylpyrrolidon-Copolymerisaten mit N-Vinylimidazol, Acryl- oder Methacrylsäure oder Gemischen hieraus als Comonomeren im Gewichtsverhältnis N-Vinylpyrrolidon zu Comonomere von 20:1 bis 1:20 oder
(d) N-Vinylimidazol-Homopolymerisaten, welche am heterocyclischen Ring durch bis zu drei C₁- bis C₄-Alkylreste substituiert sein und in durch C₁- bis C₄-Alkyl N-quaternisierter Form vorliegen können,
im Gewichtsverhältnis von 1:99 bis 90:10 mit einem Gehalt von 1 bis 35 Gew.-% an Perverbindungen.

6. C₁- bis C₄-Monopercarbonsäure- und C₄- bis C₁₈-Dipercarbonsäure-Komplexe von Mischungen aus Monosacchariden, Oligosacchariden, Stärken, Stärkeabbauprodukten oder Derivaten hiervon mit N-Vinylpyrrolidon-Homopolymerisaten im Gewichtsverhältnis von 1 : 99 bis 90 : 10 mit einem Gehalt von 1 bis 35 Gew.-% an Perverbindungen.

7. C₁- bis C₄-Monopercarbonsäure- und C₄- bis C₁₈-Dipercarbonsäure-Komplexe von Mischungen aus Trehalose, Saccharose, Glucose, α,ß- oder γ-Cyclodextrinen oder Derivaten dieser Cyclodextrine oder Gemischen hieraus mit
(a) N-Vinylcaprolactam-Homopolymerisaten,
(b) N-Vinylcaprolactam-Copolymerisaten mit N-Vinylpyrrolidon, N-Vinylimidazol, Acryl- oder Methacrylamidopropyl-3-sulfonsäure, Acryl- oder Methacrylsäure oder Gemischen hieraus als Comonomeren im Gewichtsverhältnis N-Vinylcaprolactam zu Comonomere von 20:1 bis 1:20,
(c) N-Vinylpyrrolidon-Copolymerisaten mit N-Vinylimidazol, Acryl- oder Methacrylsäure oder Gemischen hieraus als Comonomeren im Gewichtsverhältnis N-Vinylpyrrolidon zu Comonomere von 20:1 bis 1:20,
(d) N-Vinylimidazol-Homopolymerisaten, welche am heterocyclischen Ring durch bis zu drei C₁- bis C₄-Alkylreste substituiert sein und in durch C₁- bis C₄-Alkyl N-quaternisierter Form vorliegen können, oder
(e) N-Vinylpyrrolidon-Homopolymerisaten
im Gewichtsverhältnis von 1:99 bis 100:0 mit einem Gehalt von 1 bis 35 Gew.-% an Perverbindungen.

8. C₁- bis C₄-Monopercarbonsäure- und C₄- bis C₁₈-Dipercarbonsäure-Komplexe von N-Vinylpyrrolidon-Homopolymerisaten mit einem Gehalt von 1 bis 35 Gew.-% an Perverbindungen.

## Claims

1. A process for preparing C₁- to C₄-monopercarboxylic acid and C₄- to C₁₈-dipercarboxylic acid complexes in a fluidized-bed process by applying a solution of C₁- to C₄-monopercarboxylic acids, C₄- to C₁₈-dipercarboxylic acids or a mixture thereof in water or carboxylic acids to a pulverulent or pregranulated matrix in or outside the fluidized bed and simultaneous or subsequent fluidized-bed drying, wherein the matrix used is
A) an N-vinylcaprolactam homopolymer,
B) an N-vinylcaprolactam copolymer with N-vinylpyrrolidone, N-vinylimidazole, acryl- or methacrylamidopropyl-3-sulfonic acid, acrylic or methacrylic acid or a mixture thereof as comonomer in a weight ratio of N-vinylcaprolactam to comonomer of from 20:1 to 1:20,
C) an N-vinylpyrrolidone copolymer with N-vinylimidazole, acrylic or methacrylic acid or a mixture thereof as comonomer in a weight ratio of N-vinylpyrrolidone to comonomer of from 20:1 to 1:20,
D) an N-vinylimidazole homopolymer which can be substituted on the heterocyclic ring by up to three C₁- to C₄-alkyl radicals and can be N-quaternized by C₁- to C₄-alkyl,
E) a mixture of monosaccharides, oligosaccharides, polysaccharides or derivatives thereof with the polymers A to D in a weight ratio of from 1:99 to 90:10,
F) a mixture of monosaccharides, oligosaccharides, starches, starch degradation products or derivatives thereof with N-vinylpyrrolidone homopolymers in a weight ratio of from 1:99 to 90:10,
G) a mixture of trehalose, saccharose, glucose, α-, β- or γ-cyclodextrins or derivatives of these cyclodextrins or a mixture thereof with the polymers A to D or with N-vinylpyrrolidone homopolymers in a weight ratio of from 1:99 to 100:0 or
H) in the case of C₁- to C₄-monopercarboxylic acid and C₄-to C₁₈-dipercarboxylic acid complexes, an N-vinylpyrrolidone homopolymer.

2. A C₁- to C₄-monopercarboxylic acid or C₄- to C₁₈-dipercarboxylic acid complex of an N-vinylcaprolactam copolymer with N-vinylpyrrolidone, N-vinylimidazole, acryl- or methacrylamidopropyl-3-sulfonic acid, acrylic or methacrylic acid or a mixture thereof as comonomer in a weight ratio of N-vinylcaprolactam to comonomer of from 20:1 to 1:20, containing from 1 to 35% by weight of per-compounds.

3. A C₁- to C₄-monopercarboxylic acid or C₄- to C₁₈-dipercarboxylic acid complex of an N-vinylpyrrolidone copolymer with N-vinylimidazole, acrylic or methacrylic acid or a mixture thereof as comonomer in a weight ratio of N-vinylpyrrolidone to comonomer of from 20:1 to 1:20, containing from 1 to 35% by weight of per-compounds.

4. A C₁- to C₄-monopercarboxylic acid or C₄- to C₁₈-dipercarboxylic acid complex of an N-vinylimidazole homopolymer which can be substituted on the heterocyclic ring by up to three C₁- to C₄-alkyl radicals and can be N-quaternized by C₁- to C₄-alkyl, containing from 1 to 35% by weight of per-compounds.

5. A C₁- to C₄-monopercarboxylic acid or C₄- to C₁₈-dipercarboxylic acid complex of a mixture of monosaccharides, oligosaccharides, polysaccharides or derivatives thereof with
(a) an N-vinylcaprolactam homopolymer,
(b) an N-vinylcaprolactam copolymer with N-vinylpyrrolidone, N-vinylimidazole, acryl- or methacrylamidopropyl-3-sulfonic acid, acrylic or methacrylic acid or a mixture thereof as comonomer in a weight ratio of N-vinylcaprolactam to comonomer of from 20:1 to 1:20,
(c) an N-vinylpyrrolidone copolymer with N-vinylimidazole, acrylic or methacrylic acid or a mixture thereof as comonomer in a weight ratio of N-vinylpyrrolidone to comonomer of from 20:1 to 1:20 or
(d) an N-vinylimidazole homopolymer which can be substituted on the heterocyclic ring by up to three C₁- to C₄-alkyl radicals and can be N-quaternized by C₁- to C₄-alkyl,
in a weight ratio of from 1:99 to 90:10, containing from 1 to 35% by weight of per-compounds.

6. A C₁- to C₄-monopercarboxylic acid or C₄- to C₁₈-dipercarboxylic acid complex of a mixture of monosaccharides, oligosaccharides, starches, starch degradation products or derivatives thereof with an N-vinylpyrrolidone homopolymer in a weight ratio of from 1:99 to 90:10, containing from 1 to 35% by weight of per-compounds.

7. A C₁- to C₄-monopercarboxylic acid or C₄- to C₁₈-dipercarboxylic acid complex of a mixture of trehalose, saccharose, glucose, α-, β- or γ-cyclodextrins or derivatives of these cyclodextrins or a mixture thereof with
(a) an N-vinylcaprolactam homopolymer,
(b) an N-vinylcaprolactam copolymer with N-vinylpyrrolidone, N-vinylimidazole, acryl- or methacrylamidopropyl-3-sulfonic acid, acrylic or methacrylic acid or a mixture thereof as comonomer in a weight ratio of N-vinylcaprolactam to comonomer of from 20:1 to 1:20,
(c) an N-vinylpyrrolidone copolymer with N-vinylimidazole, acrylic or methacrylic acid or a mixture thereof as comonomer in a weight ratio of N-vinylpyrrolidone to comonomer of from 20:1 to 1:20,
(d) an N-vinylimidazole homopolymer which can be substituted on the heterocyclic ring by up to three C₁- to C₄-alkyl radicals and can be N-quaternized by C₁- to C₄-alkyl, or
(e) an N-vinylpyrrolidone homopolymer in a weight ratio of from 1:99 to 100:0, containing from 1 to 35% by weight of per-compounds.

8. A C₁- to C₄-monopercarboxylic acid or C₄- to C₁₈-dipercarboxylic acid complex of an N-vinylpyrrolidone homopolymer, containing from 1 to 35% by weight of per-compounds.

## Revendications

1. Procédé pour la préparation de complexes d'acides monopercarboxyliques en C1-C4 et dipercarboxyliques en C4-C18 par la technique du lit tourbillonnaire par application d'une solution d'acides monopercarboxyliqus en C1-C4, d'acides dipercarboxyliques en C4-C18 ou d'un mélange de ces acides dans l'eau ou des acides carboxyliques sur une gangue pulvérulente ou mise au préalable à l'état granuleux à l'intérieur ou à l'extérieur de la couche tourbillonnaire avec séchage simultané ou subséquent en couche tourbillonnaire, caractérisé par le fait que l'on utilise en tant que gangue
A) des homopolymères du N-vinylcaprolactame,
B) des copolymères du N-vinylcaprolactame contenant, en tant que comonomères, la N-vinylpyrrolidone, le N-vinylimidazole, l'acide acryl-ou méthacryl-amidopropyl-3-sulfonique, l'acide acrylique ou méthacrylique ou un mélange d'entre eux dans des proportions relatives en poids N-vinylcaprolactame/comonomères de 20 : 1 à 1 : 10,
C) des copolymères de la N-vinylpyrrolidone contenant, en tant que comonomères, le N-vinylimidazole, l'acide acrylique ou méthacrylique ou un mélange d'entre eux dans des proportions relatives en poids N-vinylpyrrolidone/comonomères de 20 : 1 à 1 : 20,
D) des homopolymères du N-vinylimidazole qui peuvent être substitués sur le noyau hétérocyclique par un à trois groupes alkyle en C1-C4 et peuvent être sous la forme N-quaternisée par un groupe alkyle en C1-C4,
E) des mélanges de monosaccharides, d'oligosaccharides, de polysaccharides ou leurs dérivés et des polymères A) à D) dans des proportions relatives en poids de 1 : 99 à 90 : 10,
F) des mélanges de monosaccharides, d'oligosaccharides, d'amidons, de produit de dégradation de l'amidon ou leurs dérivés et d'homopolymères de la N-vinylpyrrolidone dans des proportions relatives en poids de 1 : 99 à 90 : 10,
G) des mélanges de tréhalose, de saccharose, de glucose, d'alpha-, bêta- ou gamma-cyclodextrines ou de dérivés de ces cyclodextrines ou leurs mélanges et des polymères A) à D) ou des homopolymères de la N-vinylpyrrolidone dans des proportions relatives en poids de 1 : 99 à 100 : 0, ou bien
H) dans le cas de complexes d'acides monopercarboxyliques en C1-C4 et d'acides dipercarboxyliques en C4-C18, des homopolymères de la N-vinylpyrrolidone.

2. Complexes d'acides monopercarboxyliques en C1-C4 et d'acides dipercarboxyliques en C4-C18 et de copolymères du N-vinylcaprolactame contenant, en tant que comonomères, la N-vinylpyrrolidone, le N-vinylimidazole, l'acide acryl- ou méthacrylamidopropyl-3-sulfonique, l'acide acrylique ou méthacrylique ou leurs mélanges, dans des proportions relatives en poids N-vinylcaprolactame/comonomères de 20 : 1 à 1 : 20, à une teneur de 1 à 35 % en poids en les composés peroxydés.

3. Complexes d'acides monopercarboxyliques en C1-C4 et d'acides dipercarboxyliques en C4-C18 et de copolymères de la N-vinylpyrrolidone contenant, en tant que comonomères, le N-vinylimidazole, l'acide acrylique ou méthacrylique ou leurs mélanges, dans des proportions relatives en poids N-vinylpyrrolidone/comonomères de 20 : 1 à 1 : 20, à une teneur de 1 à 35 % en poids en les composés peroxydés.

4. Complexes d'acides monopercarboxyliques en C1-C4 et d'acides dipercarboxyliques en C4-C18 et d'homopolymères du N-vinylimidazole qui peuvent être substitués sur le noyau hétérocyclique par un à trois groupes alkyle en C1-C4 et peuvent être sous la forme N-quaternisée par un groupe en C1-C4, à une teneur de 1 à 35 % en poids en les composés peroxydés.

5. Complexes d'acides monopercarboxyliques en C1-C4 et d'acides dipercarboxyliques en C4-C18 et de mélanges de monosaccharides, d'oligosaccharides, de polysaccharides ou leurs dérivés et de
a) des homopolymères du N-vinylcaprolactame,
b) des copolymères du N-vinylcaprolactame contenant, en tant que comonomères, la N-vinylpyrrolidone, le N-vinylimidazole, l'acide acryl-ou méthacryl-amidopropyl-3-sulfonique, l'acide acrylique ou méthacrylique ou leurs mélanges, dans des proportions relatives en poids N-vinylcaprolactame/comonomères de 20 : 1 à 1 : 20,
c) des copolymères de la N-vinylpyrrolidone contenant, en tant que comonomères, le N-vinylimidazole, l'acide acrylique ou méthacrylique ou leurs mélanges dans des proportions relatives en poids N-vinylpyrrolidone/comonomères de 20 : 1 à 1 : 20 ou bien
d) des homopolymères du N-vinylimidazole qui peuvent porter sur le noyau hétérocyclique jusqu'à trois substituants alkyle en C1-C4 et peuvent être à l'état N-quaternisé par un groupe alkyle en C1-C4,
dans des proportions relatives en poids de 1 : 99 à 90 : 10 et à une teneur de 1 à 35 % en poids en les composés peroxydés.

6. Complexes d'acides monocarboxyliques en C1-C4 et d'acides dipercarboxyliques en C4-C18 et de mélanges de monosaccharides, d'oligosaccharides, d'amidons, de produits de dégradation de l'amidon ou leurs dérivés et d'homopolymères de la N-vinylpyrrolidone dans des proportions relatives en poids de 1 : 99 à 90 : 10, à une teneur de 1 à 35 % en poids en les composés peroxydés.

7. Complexes d'acides monopercarboxyliques en C1-C4 et d'acides dipercarboxyliques en C4-C18 et de mélanges de tréhalose, de saccharose, de glucose, d'alpha-, bêta- ou gamma-cyclodextrines ou de dérivés de ces cyclodextrines ou leurs mélanges, et de
a) des homopolymères du N-vinylcaprolactame,
b) des copolymères du N-vinylcaprolactame contenant, en tant que comonomères, la N-vinylpyrrolidone, le N-vinylimidazole, l'acide acryl-ou méthacryl-amidopropyl-3-sulfonique, l'acide acrylique ou méthacrylique ou leurs mélanges dans des proportions relatives en poids N-vinylcaprolactame/comonomères de 20 : 1 à 1 : 20,
c) des copolymères de la N-vinylpyrrolidone contenant, en tant que comonomères, le N-vinylimidazole, l'acide acrylique ou méthacrylique ou leurs mélanges, dans des proportions relatives en poids N-vinylpyrrolidone/comonomères de 20 : 1 à 1 : 20,
d) des homopolymères du N-vinylimidazole qui peuvent porter sur le noyau hétérocyclique jusqu'à trois substituants alkyle en C1-C4 et peuvent être à l'état N-quaternisé par un groupe alkyle en C1-C4, ou bien
e) des homopolymères de la N-vinylpyrrolidone,
dans des proportions relatives en poids de 1 : 99 à 100 : 0, avec une teneur de 1 à 35 % en poids en les composés peroxydés.

8. Complexes d'acides monopercarboxyliques en C1-C4 et d'acides dipercarboxyliques en C4-C18 et d'homopolymères de la N-vinylpyrrolidone à une teneur de 1 à 35 % en poids en les composés peroxydés.
